# EUROPEAN PATENT APPLICATION

(11) **EP 4 659 593 A1**
(43) Date of publication of application: **10.12.2025**
(21) Application number: 24201261.5
(22) Date of filing: 19.09.2024
(51) Int. Cl.: A23L 31/15, A23L 33/145, A23L 33/00, A61K 36/062, A61P 1/14, A61P 3/04, A61P 3/06, A61P 3/10, C12N 1/14

(54) **MONASCUS PILOSUS STRAIN, COMPOSITION FOR LOSING WEIGHT AND IMPROVING GUT MICROBIOTA COMPOSITION AND USE OF SAID MONASCUS PILOSUS**

(30) Priority: 07.06.2024 TW 113121392
(71) Applicant: Sunway Biotech Co., Ltd., Taipei City 114, (TW)
(72) Inventor: LEE, CHUN-LIN, 950 Taitung City, Taitung (TW); PAN, TZU-MING, 114 Taipei City (TW); PAN, CHIA YUEH, 114 Taipei City (TW); HSU, YA-WEN, 114 Taipei City (TW)
(74) Representative: Kurig, Thomas

(57) **Abstract**

The main purpose of the present invention is to provide a Monascus pilosus strain and a composition for weight loss and improvement of gut microbiota composition. The composition comprises Monascus pilosus and/or its fermented product, or comprises an active ingredient of its fermented product. Another objective of the present invention is to provide a use of Monascus pilosus in the manufacture of a composition for weight loss and improvement of gut microbiota composition. The composition of the present invention has the ability to reduce body weight and improve gut microbiota, such as lowering the ratio of Firmicutes to Bacteroidetes.

## Description

### BACKGROUND OF THE INVENTION

### 1. Field of the Invention

The present invention relates to the technical field of losing weight and improving gut microbiota, and more particularly relates to the technical field of losing weight and improving gut microbiota by the composition including Monascus pilosus, Monascus pilosus fermented products, or active ingredients of the Monascus pilosus fermented products.

### 2. Description of the Prior Art

Overweight and obesity have been recognized as one of the most neglected and troublesome health issues currently facing mankind. Even in economically prosperous developing countries, the obesity population is increasing rapidly and the obesity rate is not inferior to that of developed countries. According to the World Health Organization (WHO), overweight and obesity are defined as abnormal or excessive fat accumulation that can be detrimental to health. In addition, the WHO further defines the body mass index (BMI) equal to or greater than 25 as overweight and the BMI equal to or greater than 30 as obesity.

Although overweight and obesity are not generally considered "diseases", they are significant risk factors for non-communicable diseases, i.e., overweight and obesity can contribute to the development of cardiovascular disease (primarily heart disease and stroke), diabetes, musculoskeletal disorders (particularly osteoarthritis, a highly disabling degenerative disease of the joints), and the increase of the risk of certain cancers (including endometrial, breast, ovarian, prostate, liver, gallbladder, kidney, and colon cancers). In addition, the risk of non-communicable diseases increases with higher body mass index.

Currently, there are a variety of treatment options for overweight and obesity problems, including exercise, calorie-controlled diet, surgery, and medications. For example, physical exercise, dietary therapy, liposuction, gastrectomy, gastric bypass surgery and metabolic stimulants, appetite suppressants, starch blockers are some of the common obesity treatments available today. Among these obesity treatments, natural and healthy methods, such as dietary therapy to control calorie intake and physical exercise to burn off excess calories, are the most common ones. However, these treatments are often unsustainable due to the individual factors of the obesity patients, and the effectiveness is often unsatisfactory. Surgical procedures such as liposuction, gastrectomy or gastric bypass for weight loss have some drawbacks, such as higher costs, and the uncertainty of the procedure for obesity patients. In addition, the use of drugs such as metabolic stimulants, appetite suppressants or starch blockers may also have side effects on obesity patients, causing adverse effects on the patient's body.

In view of the limitations and drawbacks of current treatments for overweight and obesity problems, the inventor of the present invention has conducted research and experiment, and finally developed the Monascus pilosus in accordance with the present invention, which is used to reduce weight and improve the composition and the use of the gut microbiota. In daily life, obesity patients can easily lose weight and obtain obesity treatments through the easily accessible and consumable composition of the present invention, without causing adverse effects on the patient's body.

### SUMMARY OF THE INVENTION

The primary objective of the present invention is to provide a Monascus pilosus and a composition for losing weight and improving gut microbiota, the composition includes Monascus pilosus and/or its fermented product, or includes Monascinol obtained by purifying its fermented product, wherein the composition for losing weight and improving gut microbiota can be taken orally via the mouth.

To achieve the aforementioned and other objectives, the present invention provides a novel Monascus pilosus, which is deposited at the National Collections of Industrial, Food and Marine Bacteria (NCIMB Ltd) in the United Kingdom under the Accession Number of NCIMB 44103.

Further, the present invention provides a composition for losing weight and improving gut microbiota, which contains a Monascus pilosus fermented product with an effective amount, wherein the Monascus pilosus fermented product is prepared by fermenting a base material by a Monascus pilosus, and the Monascus pilosus is deposited at the National Collections of Industrial, Food and Marine Bacteria (NCIMB Ltd) under the Accession Number NCIMB 44103. In addition, the composition of the present invention also may contain a pharmaceutically acceptable vehicle.

In the present invention, the composition for losing weight and improving gut microbiota has the ability of enhancing the bacterial abundance of Bacteroidales thereby lowering the ratio of Firmicutes to Bacteroidetes.

In the present invention, the composition for losing weight and improving gut microbiota composition further has the ability of enhancing the bacterial abundance of beneficial intestinal flora such as Verrucomicrobiales and Bifidobacteriales. In addition, the composition has the effects of enhancing the bacterial abundance of beneficial intestinal flora and changing gut microbiota composition.

In the present invention, the base material of the composition is a rice, dioscoreae rhizome (yam), or mixture of related carbohydrates.

In addition, the effective amount of the composition of the present invention is at least 0.5g per day of the Monascus pilosus fermented product for adults, which contains 1.5 mg Monascinol, wherein the Monascus pilosus fermented product contains at least one active ingredient, and the active ingredient contains at least one selected from the group consisting of Monascinol, Ankaflavin and Monascin.

In the present invention, the composition can be a food composition, a pharmaceutical composition, a feed composition, a nutritional supplement composition, a dietary supplement composition, or a food additive composition.

The present invention further provides a composition for losing weight and improving gut microbiota, which contains an active ingredient with an effective amount, and the active ingredient is extracted from a Monascus pilosus fermented product, wherein the Monascus pilosus fermented product is prepared by fermenting a base material by a Monascus pilosus, and the Monascus pilosus is deposited at the National Collections of Industrial, Food and Marine Bacteria (NCIMB Ltd) under the Accession Number of NCIMB 44103. In addition, the composition of the present invention also may contain a pharmaceutically acceptable vehicle.

In the present invention, the composition for losing weight and improving gut microbiota composition has the ability of enhancing the bacterial abundance of Bacteroidales, thereby lowering the ratio of Firmicutes to Bacteroidetes.

In addition, in the present invention, the composition for losing weight and improving gut microbiota has the ability of enhancing the abundance of beneficial intestinal flora such as Verrucomicrobiales and Bifidobacteriales. Furthermore, the composition has the ability of enhancing the abundance of beneficial intestinal flora and changing the composition of gut microbiota.

In the composition of the present invention, the active ingredient contains at least one selected from the group consisting of Monascinol, Ankaflavin and Monascin. In the composition of the present invention, the base material is a rice, dioscoreae rhizome (yam), or mixture of related carbohydrates, the effective amount is at least 0.75 mg to 12 mg of the active ingredient or at least 3 mg of the active ingredient per day for adults.

Further, the present invention provides a use of a Monascus pilosus for preparing a composition for losing weight and improving gut microbiota, wherein the Monascus pilosus is deposited at the National Collections of Industrial, Food and Marine Bacteria (NCIMB Ltd) under the Accession Number of NCIMB 44103.

In addition, the present invention also provides an application of a composition for losing weight and improving the composition of gut microbiota. This type of application includes food, beverages, health food, additives, pharmaceutical compositions and other forms that are easy to use in daily life for ordinary people to achieve daily intestinal health, and to achieve weight loss and fat loss to avoid diseases such as cardiovascular disease, diabetes, musculoskeletal diseases and cancer caused by overweight and obesity.

### BRIEF DESCRIPTION OF THE DRAWINGS

FIG. 1 shows a flow chart of the identification of a Monascus pilosus strain of the present invention;
FIG. 2 shows a phylogenetic tree of the Monascus pilosus of the present invention after the alignment of the β-tubulin sequence;
FIG. 3 shows a phylogenetic tree of the Monascus pilosus of the present invention after the alignment of the ITS sequence;
FIG. 4 shows a species-specific PCR analysis result of the Monascus pilosus of the present invention;
FIG. 5 shows a PCR analysis result of pksCT genes of the Monascus pilosus of the present invention;
FIG. 6 shows the morphology of an ascocarp of the Monascus pilosus of the present invention;
FIG. 7A shows an analysis result of the bacterial abundance in the phylum-level classification of high-fat diet rats fed with red yeast rice (RL: 0.5 g/day and RH: 2 g/day) and Monascinol (MSol: 3 mg/day) in accordance with the present invention;
FIG. 7B shows an analysis result of the bacterial abundance in the class-level classification of high-fat diet rats fed with red yeast rice (RL: 0.5 g/day and RH: 2 g/day) and Monascinol (MSol: 3 mg/day) in accordance with the present invention;
FIG. 7C shows an analysis result of the bacterial abundance in the order-level classification of high-fat diet rats fed with red yeast rice (RL: 0.5 g/day and RH: 2 g/day) and Monascinol (MSol: 3 mg/day) in accordance with the present invention;
FIG. 7D shows an analysis result of the bacterial abundance in the family-level classification of high-fat diet rats fed with red yeast rice (RL: 0.5 g/day and RH: 2 g/day) and Monascinol (MSol: 3 mg/day) in accordance with the present invention;
FIG. 7E shows an analysis result of the bacterial abundance in the genus-level classification of high-fat diet rats fed with red yeast rice (RL: 0.5 g/day and RH: 2 g/day) and Monascinol (MSol: 3 mg/day) in accordance with the present invention;
FIG. 7F shows an analysis result of the bacterial abundance in the species-level classification of high-fat diet rats fed with red yeast rice (RL: 0.5 g/day and RH: 2 g/day) and Monascinol (MSol: 3 mg/day) in accordance with the present invention;
FIG. 8 shows the relative abundance of key bacterial groups in the rats of each experimental group (NOR: normal control group, RL, RH and MSol) compared with the rats of a high-fat diet group (HF) in the order-level classification; and
FIGS. 9A~9D shows a chart of the relative abundance of bacterial community aggregation in each experimental group (NOR, HF, RL, RH and MSol).

### DETAILED DESCRIPTION OF THE PREFERRED EMBODIMENTS

All technical and scientific terms used in the present invention, unless otherwise defined, have the meaning commonly understood by persons of ordinary skill in the art to which the present invention pertains. The present invention is illustrated by the following examples, which are illustrative and not limiting, and the present invention is not subject to the limitations of the following examples. Unless otherwise noted, the materials used in the present invention are commercially available, and the following are only examples of available channels.

The present invention provides a novel Monascus pilosus, which is deposited at the National Collections of Industrial, Food and Marine Bacteria (NCIMB Ltd) under the Accession Number of NCIMB 44103. In addition, the present invention provides a composition for losing weight and improving gut microbiota, which contains a Monascus pilosus fermented product with an effective amount, and the Monascus pilosus fermented product is prepared by fermenting that using the Monascus pilosus deposited at the National Collections of Industrial, Food and Marine Bacteria (NCIMB Ltd) under the Accession Number of NCIMB 44103. Further, the present invention provides a composition for losing weight and improving gut microbiota, which contains an active ingredient with an effective amount, and the active ingredient is extracted from a Monascus pilosus fermented product, wherein the Monascus pilosus fermented product is prepared by fermenting a base material by the Monascus pilosus deposited at the National Collections of Industrial, Food and Marine Bacteria (NCIMB Ltd) under the Accession Number of NCIMB 44103. In addition, the active ingredient is Monascinol, and the base material is a rice, dioscoreae rhizoma, or mixture of related carbohydrates. Further, in the present invention, experiments are conducted with obesity rats induced by high-fat feed, and it is confirmed that Monascus pilosus and the composition prepared thereof in accordance with the present invention have the functions of losing weight and improving gut microbiota.

The Monascus pilosus strain of the present invention also includes successive generations or mutant strains, that have the same stain characteristics, genomic characteristics, or uses (for weight loss and improvement of the gut microbiota) as those described in the present invention.

The compositions described herein may include but not limited to food, beverage, health food, animal drink additive, animal feed additive, animal and human pharmaceutical composition, food additive, beverage additive, etc. that are applicable to the present invention.

The term "losing weight" refers to the circumstance that the composition of the present invention is effective to reduce body weight as compared to a person who does not use a composition comprising Monascus pilosus of the present invention, or a fermented product thereof, or an extract of a fermented product thereof. The term "improving" refers to the circumstance that the composition of the present invention is effective in improving the gut microbiota as compared to a person who does not use a composition comprising Monascus pilosus of the present invention, or a fermented product thereof, or an extract of a fermented product thereof.

The term "effective amount" refers to the effective amount that can effectively lose weight and improve the gut microbiota, or can be called "effective amount for treatment" or "effective amount for improvement". The term "pharmaceutically acceptable" refers to substances or compositions that must be compatible with the other components of the prepared substances and not be harmful to patients.

The composition of the present invention can be prepared using techniques well known to those skilled in the art. The Monascus pilosus, its fermented product or the extract of its fermented product, and a pharmaceutically acceptable vehicle are used for preparing the composition in a dosage form suitable for the present invention, wherein the dosage form includes but not limited to solution, emulsion, suspension, powder, tablet, pill, lozenge, troche, capsule or other similar or suitable dosage form for the present invention.

In the aforementioned composition, one or more dissolution aids, buffers, preservatives, colorants, spices, flavors, excipients, etc. commonly used in the field of preparations may also be appropriately added as needed.

In another preferred embodiment, the aforementioned composition provided by the present invention is further added into an edible material to prepare a food product or a health product; wherein the edible material includes but is not limited to: water; fluid milk product; milk; concentrated milk; fermented milk, such as yogurt, frozen yogurt, sour milk, lactic fermenting beverage; milk powder; ice cream; cream cheese; dry cheese; soybean milk; fermented soybean milk; fruit and vegetable juice; juice; sports drink; confectionery; jelly; baby food; health food; animal feed; herbal medicine; dietary supplement, etc.

Further, the present invention also provides a method for reducing weight and improving gut microbiota, which is to use the aforementioned composition with the effective amount for overweight and obesity patients to reduce weight and improve their gut microbiota.

In addition, the present invention further provides the method or use of preparing the composition of the Monascus pilosus, Monascus pilosus fermented product or the extract of the Monascus pilosus fermented product for the purpose of improving the composition of gut microbiota.

The route of administration of the composition for losing weight and improving gut microbiota provided by the present invention can be appropriately adjusted according to the needs, but it is not particularly limited, and the preferred route of administration is oral administration in an appropriate dosage form.

### Embodiment 1: Strain identification of a novel Monascus pilosus in accordance with the present invention

### 1. Basis and Process of the Strain Identification

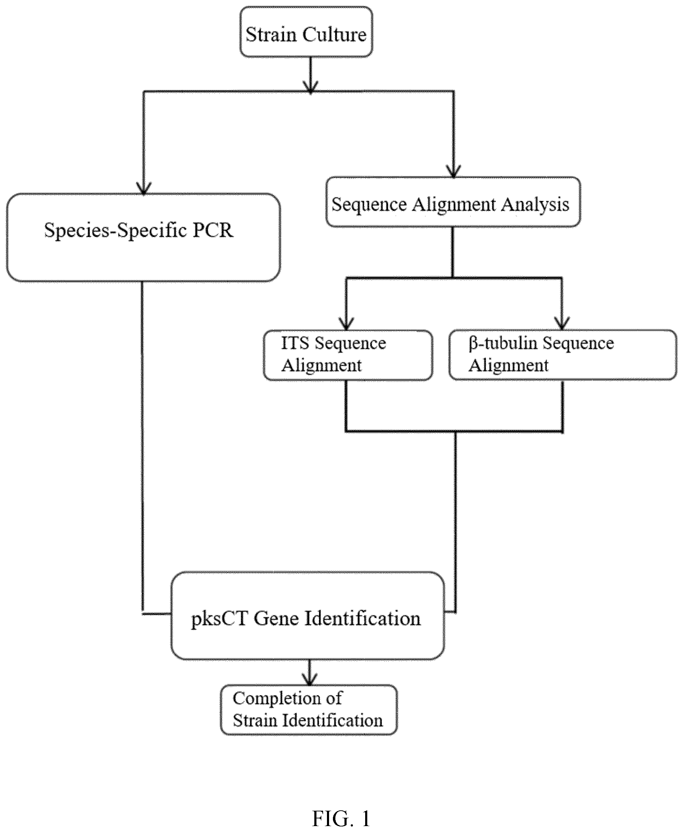

The traditional classification and identification of Monascus pilosus is mainly based on colony morphology, of which colony size and color are the main bases (Hawksworth and Pitt, 1983). However, some Monascus pilosus strains (such as the Monascus pilosus and Monascus ruber) are not easy to classify based on colony appearance. Therefore, molecular subtyping methods are used for the species identification of Monascus pilosus. The molecular subtyping method of Monascus pilosus is currently based on sequence alignment of β-tubulin and ITS (Park et al., 2004). However, in order to further improve the accuracy of strain identification, the present invention, in addition to the use of sequence alignment of β-tubulin and ITS, also uses the strain-specific polymerase chain reaction (PCR) and pksCT gene PCR developed by the inventor's laboratory to further improve the accuracy of strain identification (see FIG. 1).

### 2. Strain Culture and Deoxyribonucleic Acid (DNA) Extraction

Potato dextrose broth (PDB) medium was purchased from Difco (Becton-Dickinson Diagnostic System, Sparks, MD, USA). For DNA extraction samples, the strains to be tested were cultured in PDB medium at 28°C for 7 days, collected and ground into powder in liquid nitrogen, and dried in a vacuum oven. Then, 0.1 g of dry bacterial powder was weighed and put into a 2-mL microcentrifuge tube, and the QIAamp DNA Mini Kit (purchased from QIAGEN N.V, Velno, The Netherlands) was used for DNA extraction.

### 3. ITS, β-tubulin Sequence Alignment Analysis and Species-Specific PCR Analysis (1) ITS Sequence PCR Amplification

The total volume of the PCR reaction was 25 µL, which contained 1x PCR buffer, 0.05 mM of four kinds of deoxynucleoside triphosphates (dNTPs), 5 U ExSel high fidelity DNA polymerase (purchased from Bertec Enterprise Co., Ltd., Taipei), 0.2 µM of primer ITS 1 (Sequence ID Number 1) and primer ITS4 (Sequence ID Number 2) and 0.16 µg of template DNA. The PCR reaction conditions included an initial denaturation performed at 95°C for 5 min., and then the denaturations at 95°C for 30 sec., at 62°C for 30 sec. and at 70°C for 1 min, which was defined as one cycle, and 35 cycles were carried out. Finally, a denaturation was performed at 70°C for 10 min. before the product was stored at 4°C. The product was confirmed by electrophoresis and then submitted to Genomics BioSci & Tech, Co. Ltd. (Taipei, Taiwan) for DNA sequencing.

### (2) β-tubulin Sequence PCR Amplification

The total volume of the PCR reaction was 25 µL, which contained 1x PCR buffer, 0.05 mM of dNTPs, 5 U of Exsel high fidelity DNA polymerase, 0.12 µM of primer β-tubulin F (Sequence ID Number 3) and primer β-tubulin R (Sequence ID Number 4) (Park et al., 2004) and 0.16 µg of template DNA. The PCR reaction conditions includes an initial denaturation performed at 95°C for 5 min., and then denaturations at 95°C for 30 second, at 55°C for 2 min., at 70°C for 2 min. which was defined as one cycle, and 35 cycles were carried out. Finally, a denaturation was performed at 70°C for 10 min. before the product was stored at 4°C. The product was confirmed by electrophoresis and then submitted to Genomics BioSci & Tech, Co. Ltd. (Taipei, Taiwan) for DNA sequencing.

### (3) Monascus purpureus (M. purpureus) Species-Specific PCR

M. purpureus species-specific PCR was designed based on the residual fragment of the mokH monacolin K biosynthetic gene unique to M. purpureus genomic, and capable of effectively identifying whether Monascus pilosus belongs to M. purpureus. The total volume of the PCR reaction was 25 µL, which contained 1x PCR buffer, 0.05 of mM dNTPs, 5 U of DNA polymerase (SupeTherm GOLD DNA polymerase), 0.12 µM of primer MPuS 1 (Sequence ID Number 5) and primer MPuS2 (Sequence ID Number 6) and 0.16 µg of template DNA. The PCR reaction conditions included an initial denaturation performed at 95°C, 10 min., and then denaturations at 95°C for 30 sec. and at 60°C for 1 min. which was defined as one cycle, and 35 cycles were carried out. Finally, a denaturation was performed at 70°C for 10 min. before performing the electrophoresis analysis.

### (4) Monascus pilosus/ruber (M. pilosus/rube) Species-Specific PCR

M. pilosus/ruber species-specific PCR was designed based on the conserved fragment of the FAS gene in the M. ruber genomic pigment biosynthesis gene cluster, and capable of effectively identifying whether Monascus pilosus belongs to M. pilosus/ruber. The total volume of the PCR reaction was 25 µL, which contained 1x PCR buffer, 0.05 of mM dNTPs, 5 U of SupeTherm GOLD DNA polymerase, 0.12µM of primer RubPil F (Sequence ID Number 7) and primer RubPil R (Sequence ID Number 8) and 0.16 µg of template DNA. The PCR reaction conditions included an initial denaturation performed at 95°C for 10 min., and then denaturations at 95°C for 30 sec., and at 60°C for 1 min. which was defined as one cycle, and 35 cycles were performed. Finally, a denaturation was performed at 70°C for 10 min. before performing the electrophoresis analysis.

### (5) pksCT Genes PCR

The pksCT gene is the core gene for the production and synthesis of citrinin in M. purpureus, these genes have been lost in the genomic of M. pilosus/ruber, so that they can be used as an auxiliary determination basis for the identification of M. pilosus/ruber. The total volume of the PCR reaction was 25 µL, which contained 1x PCR buffer, 0.05 mM of dNTPs, 5 U of SupeTherm GOLD DNA polymerase, 80 nM of primer pksCT-M R (Sequence ID Number 9) and primer pksCT-M F (Sequence ID Number 10) and 10 ng of template DNA. The PCR reaction conditions included an initial denaturation performed at 95°C for 10 min., and then denaturations performed at 95°C for 30 sec., at 54°C for 30 sec., at 72°C for 40 sec., which was defined as one cycle, and 30 cycles were performed. Finally, a denaturation was carried at 70°C for 10 min. before performing the electrophoresis analysis.

### (6) Sequence Alignment Analysis

Geneious 8.1.9 software (by Biomatters Ltd., Auckland, New Zealand) was used for the sequence alignment analysis. Geneious built-in assembler was used for sequence combination, MAFFT 7.017 was used for multiple alignment, MEGA was used for establishing the phylogenetic tree of maximum likelihood, the nucleic acid substitution mode was General Time Reversible (GTR), and bootstrapping was performed for 1000 times. MrBayes was used for Bayesian phylogenetic tree and post hoc probability test, the nucleic acid substitution mode was GTR, and Aspergillus terreus was used as an out group.

### ITS and β-tubulin Sequence Alignment Analysis Results

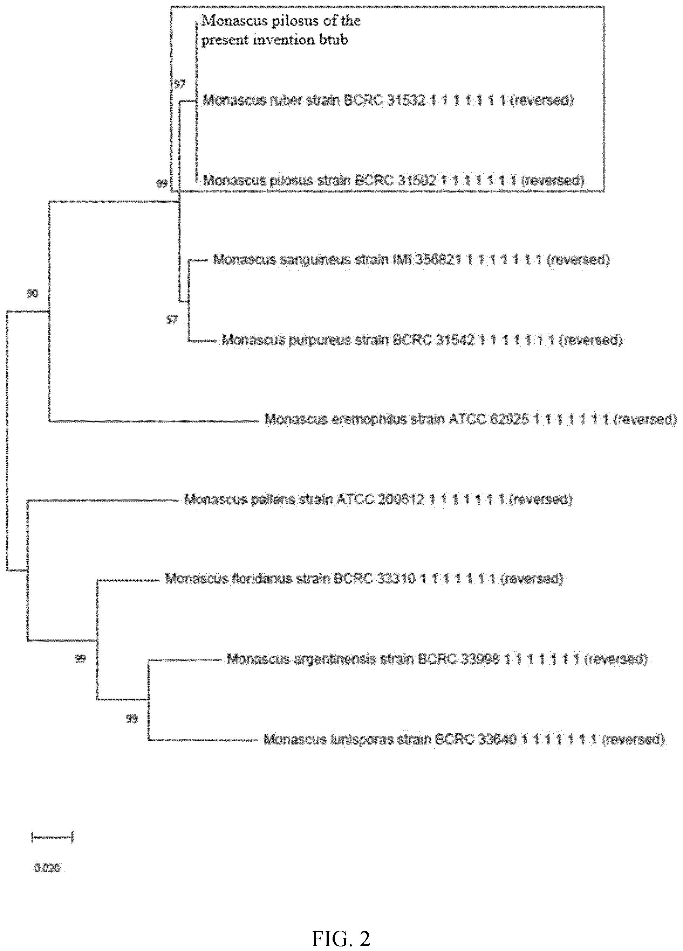

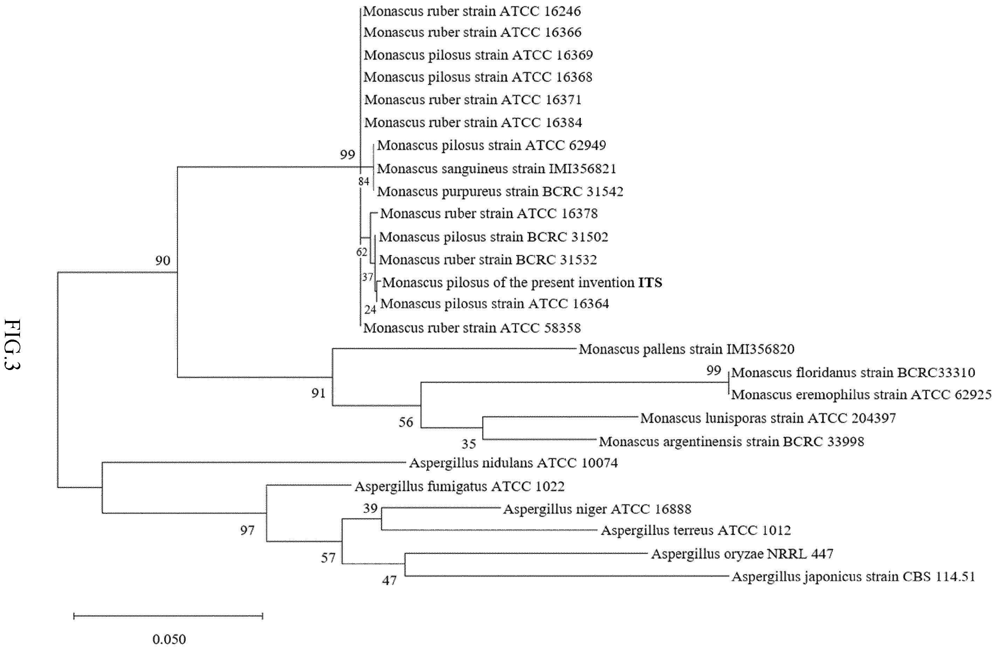

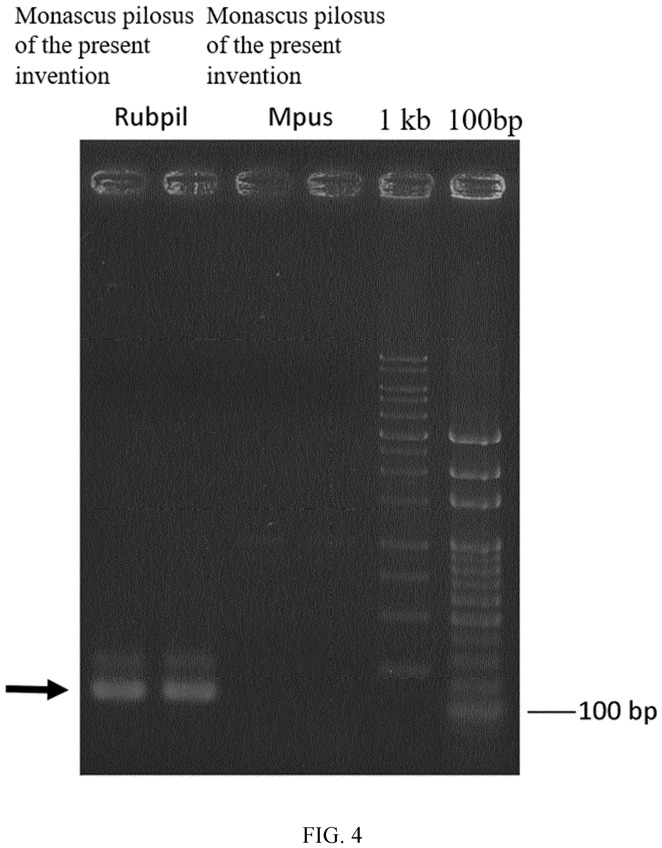

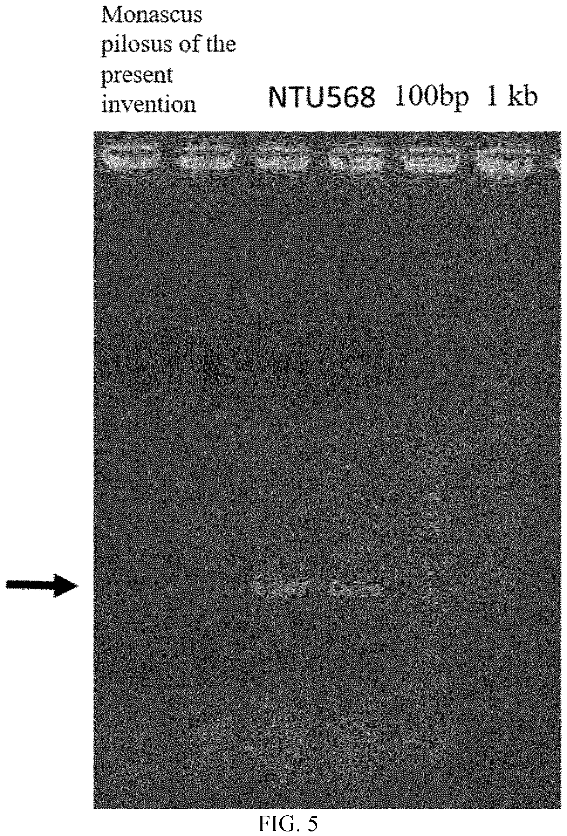

After the sequences of β-tubulin and ITS of the Monascus pilosus of the present invention were aligned, their phylogenetic trees were as shown in FIGS. 2 and 3 respectively. The sequence alignment result of β-tubulin indicated that the Monascus pilosus of the present invention was of the same branch of M. pilosus and M. ruber, with a permutation support of 97% (>50%). The sequence alignment result of ITS indicated that the Monascus pilosus of the present invention was of the same monophyletic group with the branch of M. purpureus, with a permutation support of 62% (>50%) (See FIG. 3). The results shown in FIGS. 2 and 3 consistently indicated that the Monascus pilosus of the present invention belonged to either the species of M. pilosus or M. ruber. Species-specific PCR analysis results were consistent with the phylogenetic tree analysis results (See FIG. 4, where RubPil is M. ruber/pilosus-specific, and Mpus is M. purpureus-specific). The pksCT gene PCR results also indicated that the genomic of the Monascus pilosus of the present invention did not contain pksCT genes (See FIG. 5, where NTU 568 Monascus purpureus is the positive control), belonging to either the species of M. pilosus or M. ruber.

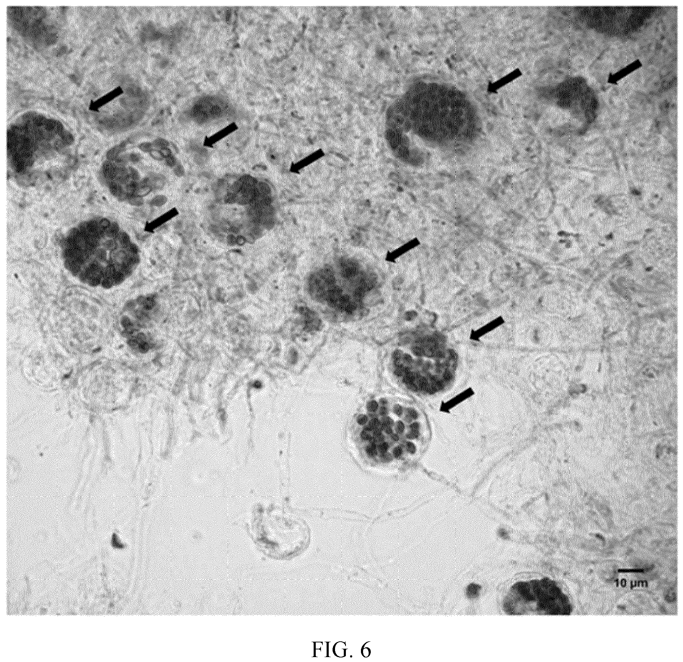

In addition, the current classification for distinguishing M. pilosus and M. ruber resides on whether or not the shell of ascocarp has pigment (which is usually brown for M. ruber and colorless for M. pilosus). The results indicated that the ascocarps of the Monascus pilosus of the present invention were colorless (See FIG. 6). Therefore, the species of the Monascus pilosus of the present invention was determined to be M. pilosus.

Therefore, in the first embodiment as described above, the results of the ITS sequence alignment, β-tubulin sequence alignment, strain(species)-specific PCR and pksCT gene PCR were all consistent, showing that the Monascus pilosus of the present invention belonged to either the species of M. pilosus or M. ruber. After the morphological observation of ascocarps, the Monascus pilosus of the present invention was determined to be the species of M. pilosus. In addition, we can also know from the above sequence alignment analysis results of ITS and β-tubulin that the Monascus pilosus of the present invention is a novel Monascus pilosus isolate.

### Embodiment 2: Culture Method of the Monascus pilosus of the Present Invention

### 1. Inoculum Culture:

(1) Weigh 2 g of rice flour and place it into a 500-mL triangular culture flask with a linear groove at the bottom. Add 100 mL of reverse osmosis water (RO water), plug a breathable silicone stopper to the opening of the flask and shake well. Sterilize at 1.25 Atmospheric pressure (atm), 121°C for 20 min., and after sterilization, cool the flask at room temperature for later use.
(2) Dig out 3 small pieces of Monascus pilosus inoculum from the petri dish and place them into a 500-mL flask, plug the flask with a breathable silicone stopper, and culture it with shaking in a 30°C incubator at 150~200 rpm for 48 - 72 hours.

### 2. Solid State Culture

(1) Inoculation: Soak 300 g of rice in RO water overnight, drain the water, wrap it in koji cloth, place it in a koji dish, sterilize (at 121°C for 30 min) and then cool for inoculation. Take out the Monascus pilosus inoculum liquid and inoculate it into the solid base material (10%) and mix thoroughly.
(2) The culture steps are as follows:
   a. Culture Conditions: After inoculation, wrap it with koji cloth properly, and put it in a constant temperature and humidity room at a temperature of 30°C and a relative humidity 60% for culture.
   b. Water Replenishment: When the Monascus pilosus reproduces rapidly, some of the water in the solid base material will be evaporated due to the rising temperature and most will be consumed by reproduction. Therefore, the base material becomes dry and needs to be replenished with water. Replenish about 30~50 mL of sterile water daily from the 2nd day to the 11th day, and then perform water replenishment every other day until the collection of Monascus pilosus.
   c. Collection of Monascus pilosus: Place the Monascus pilosus fermented product that has been cultured for 21 days (culture for 14-28 days) in an oven to dry (at 37°C for 24 hr), and then store it after drying, and this Monascus pilosus fermented product is red yeast rice.

### Embodiment 3: Monascinol Purification and Separation Method

1. Weight about 1000 g of red yeast rice.
2. Add about 10 liters (L) of 95% EtOH and perform an extraction in a water bath at 60°C. Shake evenly every 30 minutes, and continue the extraction for 2 hours. Filter the extract with filter paper and repeat the extraction once.
3. Concentrate the filtered extract to a thick form under reduced pressure, add 2 to 2.5 times the weight of silica gel, mix and concentrate them to dryness under reduced pressure, freeze-dry them in a freeze dryer overnight before weighing.
4. Weigh about 7 to 8 times the weight of the silica gel from Step 3 and mix with Hex:EtOAc (8:2) solvent to fill into an open chromatography column. After the chromatography column is balanced, add the mixture of silica gel and extract from Step 3 above. Spread the mixture flatly on top and waits for washing.
5. Wash the chromatography column sequentially with Hex:EtOAc (8:2), Hex: EtOAc (7.5:2.5), Hex:EtOAc (7:3), and Hex:EtOAc (6:4). Collect the Hex: EtOAc (7:3) eluent.
6. Concentrate the Hex:EtOAc (7:3) eluent to dryness under reduced pressure and then redissolve it in MeOH, and finally purify it using preparative HPLC. The solvent conditions are MeOH:ddH2O (83:17), collect the yellow pigment eluent of the Monascus pilosus and concentrate it to dryness under reduced pressure, and finally remove the residual moisture by vacuum drying to obtain pure Monascinol.

### Embodiment 4: The Method for Conducting Experiments on Obesity Rats Induced by High-fat Feed Using the Composition of the Present Invention

### 1. Preparation of the Composition of the Present invention

(1) Msol Group: This group used Monascinol with an adult daily dosage of 3 mg/day.
(2) RL and RH Groups: These groups used the red yeast rice obtained by fermenting the Monascus pilosus fermented rice of the present invention and the red yeast rice contained 3 mg/g of Monascinol, wherein the adult daily dosage of the red yeast rice for the RL and RH groups were 0.5 g/day and 2 g/day respectively.

### 2. Experiment on Evaluation of Adjustment of Body Fat Composition

### (1) Animal Feeding and Care

Forty male rats of 8-week-old Sprague-Dawley (SD) strain were purchased from BioLASCO (Taipei, Taiwan) for the experiment. The growing environment was one with constant indoor temperature (25 ± 1°C), relative humidity of 60%, and 12 hours of cyclic light (light time from 8:00 to 20:00). The rats were acclimatized for four days before the formal experiment, then reared in 5 groups of 8 rats each for 8 weeks and were fed in an ad libitum feeding manner, and their body weight, food intake and water intake were measured at regular intervals during the experimental period.

### (2) Animal Feeding Dosage Conversion

The feeding dosage was calculated according to the method of "Estimating the maximum safe starting dose in initial clinical trials for therapeutics in adult healthy volunteers" published by the U.S. Food and Drug Administration in 2005. Provided that an adult of 60 kg was used as the base, the principle of dosage conversion is 6.2 times of the recommended daily intake of mg/kg bw/day for human as the recommended daily intake of mg/kg bw/day for rat, and the rat group is configured and fed with the dosage as shown in Table 1. The formulae are as follows: Dose intake per kilogram of body weight in rats = Recommended intake in humans ÷ Body weight 60 kg × 6.2, and thus the daily dose intake per kilogram of body weight in rats can be calculated.

### (3) Grouping and Induction of Experimental Animal

The experimental rats were divided into groups, and the obesity rats were induced by high-fat feed as follows (Please refer to Table 1).
i. NOR Group: Normal animal group.
ii. HF Group: High-fat control group including obesity rats induced by high-fat feed.
iii. RL Group: Obesity rats induced by high-fat feed and tube-fed daily with red yeast rice powder, equivalent to 0.5 g of red yeast rice containing 1.5 mg of Monascinol per day consumed by an adult rat of 60 kg.
iv. RH Group: Obesity rats induced by high-fat feed and tube-fed red yeast rice powder daily, equivalent to 2 g of red yeast rice containing 6 mg of Monascinol per day consumed by an adult of 60 kg.
v. Msol Group: Obesity rats induced by high-fat feed and tube-fed Monascinol powder daily, equivalent to 3 mg of Monascinol powder per day consumed by an adult of 60 kg.

**Table 1. Experimental Dose Given and Grouping Design**

| Experimental Group | Feed and Drink^{a} | Substance Delivered | Dosage (mg/kg BW) | Relative Human Dosage (mg/60kg BW) |
|---|---|---|---|---|
| NOR | Normal Diet | RO Water | - | - |
| HF | High Fat Diet | RO Water | - | - |
| RL | High Fat Diet | Red yeast rice powder | 51.67 | 500 |
| RH | High Fat Diet | Red yeast rice powder | 206.67 | 2000 |
| Msol | High Fat Diet | Monascinol | 0.31 | 3 |

| | | | | |
|---|---|---|---|---|
| ^{a} The test substance was fed for 8 weeks. Normal diet was 100% chow diet; high fat-diet feed consisted of 72.3% chow diet, 26.7% ghee powder and 1% cholesterol. | | | | |

(4) Test Substance Feeding Method. The test substance was administered orally by tube feeding, and the concentration of the test substance was adjusted by using reverse osmosis water. 2.0 mL of sterile plastic syringes with stainless steel feeding pins were used to feed each group of test substance daily. Stainless steel feeding needles were soaked in 70% alcohol solution and rinsed with sterile distilled water several times before use. Each group of test animals was administered the test substance once a day for 8 weeks.

(5) Sacrificial Blood Sampling: At the 8^{th} week, the rats fasted for 12-14 hours before blood sampling. All rats were euthanized with carbon dioxide and blood samples were collected by inferior vena cava sampling. Blood samples were centrifuged with the conditions of 4°C and 3,000 x g for 15 min.

### (6) Test Substance Delivery Method

After four days of acclimatization, the rats were divided into groups. The NOR group was fed with reverse osmosis water as drinking water and Lab diet 5001 (chow diet) with an energy content of 3.35 kilocalories per gram (kcal/g), while the remaining four groups were induced with a high-fat (obesity) method. The high-fat feed was a high calorie feed (4.31 kcal/g) containing 72.3% LabDiet 5001 powder, 26.7% butter powder, and 1% cholesterol for obesity induction.

### (7) Evaluation of Body Fat Composition Indexes

### i. Change of Body Weight

The body weight of rats was measured weekly and the changes in body weight of each group were compared at the end of the experiment.

### ii. Body Fat Mass and Body Fat Rate

At the time of sacrifice, fatty tissues surrounding the kidneys and the epididymis (perirenal and epididymal fatty tissues) of the experimental animals were removed, and the their weights were measured and recorded. The body fat rate was calculated as follows: Body fat efficiency = (fat weight / body weight) × 100%

### (8) Animal Sacrifice and Sample Preparation

The animals fasted for 16 hours before sacrifice, and carbon dioxide was used for asphyxiation during sacrifice. After making sure that there was no heartbeat, blood was drawn and organs were collected. Blood was drawn from the rat's inferior vena cava with a needle. After blood collection, the needle was slowly withdrawn from the blood vessel and the blood was placed into a 2 mL eppendorf tube and centrifuged at 10,000 x g for 10 min. The upper serum layer was separated into eppendorf tubes and stored in a -20°C freezer for future analysis.

### (9) Serum Biochemical Analysis

The test was performed using a biochemical autoanalyzer (Model No.: Beckman-700, purchased from Fullerton, California, USA). The items measured were Total Cholesterol (TC), and Triglyceride (TG).

### (10) Liver and Fecal Lipid Extraction and Determination

0.1 g of liver tissue or fecal powder was weighed and 1 mL of chloroform: methanol (2: 1, v/v volume ratio) was added, and homogenized with a tissue homogenizer and centrifuged to obtain the supernatant. The supernatant was then air-dried to remove the solvent, and the sample was dissolved by adding dimethyl sulfoxide (DMSO), and the extracts were kept at -20°C. The TC concentration was analyzed using a commercially available biochemical reagent (Model No. BXC 0261, Fortress), and the TG concentration was analyzed using a commercially available biochemical reagent (Model No. BXC 0271, Fortress), as per the kit instruction manual.

### (11) Biostatistical Analysis Method

The experimental results were presented as mean ± standard deviation (mean ± SD). Statistical Package for the Social Sciences (SPSS 12.0) system's one-way ANOVA was used for statistical processing, and Duncan's test was used to compare the differences between groups, where p < 0.05 indicated a significant difference.

### (12) Gut Microbiota Research Method

### i. Gut Microbiota Fecal Analysis

### Fecal Sample Collection

About 0.3 g of fresh feces was collected into 1.5 mL of eppendorf before fasting for sacrifice, sealed with parafilm, stored at -80°C for analysis, and sent to BioTools (New Taipei, Taiwan) for analysis.

### ii. Genomic DNA Extraction and PCR Amplification and Purification

Total genomic DNA (QIAamp PowerFecal DNA, Qiagen) was extracted from the samples. The DNA concentration was determined by Qubit 4.0 fluorometer (Thermo Scientific) and adjusted to 1 ng/µL. The full-length 16S gene (V1-V9 region) was amplified by 16S gene-specific primers with barcodes (Forward primer 1): 5' Phos/ GCATC-16-base barcode -Sequence ID Number 11, Reverse primer 1: 5'Phos/GCATC- 16-base barcode - Sequence ID Number 12). PCR reactions were performed using KAPA HiFi HotStart Ready Mix (Roche): at 95°C for 3 min. and 20-27 cycles (Sample Dependency): at 95°C for 30 sec., at 57°C for 30 sec., at 72°C for 60 sec; at 72°C for 5 min., and finally stored at 4°C for future use. PCR products were monitored on 1% agarose gels. Samples of approximately 1500 bp bright bands were selected and purified by using AMPure PB Beads for SMRTbell library preparation.

### iii. Construction and Sequencing of SMRTbell Library

SMRTbell libraries were prepared based on full-length 16S gene amplifications with barcoded primers used in the Multiplex SMRTbell Library Preparation and Sequencing Program (Pacbio). Sequencing was performed in circular consensus sequencing (CCS) mode on a PacBio Sequel IIe instrument, generating HiFi read with a Phred Scale = 30. The DNA to be tested will be sequenced iteratively. By comparing the results of the reverse sequencing, sequencing errors will be corrected, ultimately achieving a high accuracy of >99.9% (QV30).

### iv. Bioinformatics Analysis

Information Analysis (Sequential data processing/denoising and species annotation) The sample data from the downstream data were sorted out according to the Barcode sequence and PCR amplification primer sequences. After the removal of the barcodes and primer sequences, the data were imported into QIIME2 (v2019.7.0; https://qiime2.org/) and all the sequences of the samples were saved into one corresponding object Artifacts. QIIME2 cutadapt was used to remove the amplicon variant primers (forward primer 2: Sequence ID Number 13; reverse primer 2: Sequence ID Number 14), and the sequence with the primers removed will be used as QIIME2 DADA2. Input Reads.

The denoising analysis began with the filtering of sequences, setting each sequence to be cropped to a specified length (forward reads: 280 bp; reverse reads: 220 bp) and setting the MaxEE (forward reads: 2; reverse reads: 2) parameter as the maximum number of expected error bases allowed for the sequence. This was followed by denoising, the core algorithm of DADA2 utilizing the information of sequence abundance, quality score, and relationships between sequences to correct the alkaloid of sequencing errors and to infer the real sequences. Splicing was performed after denoising each end of the sequence (minimum overlap length 20 bp; overlap region was not allowed to be mismatched). Finally, the spliced sequences are subjected to chimera removal by comparing the sequences with higher abundance in the sample. If a sequence with low abundance is similar to several sequences, it can be recognized as a chimera and removed from the sample. Sequences after denoising analysis were used as representative sequences of Amplicon Sequence Variants (ASVs) and can be obtained from the ASV table for subsequent species annotation. The QIIME2 feature-classifier (v2019.7.0; https://qiime2.org/) was used to select machine learning-based classification methods and to train naive bayes classifiers for different databases and specific variability regions. The taxonomic annotation of ASVs represented by this classifier yields taxonomic information and the composition of the microbial phyla of each sample at each classification level including kingdom, phylum, class, order, family, genus, and species.

### v. Statistical Analysis of Species Differences

Welch's t-test was analyzed by STAMP (v2.1.3), and the intergroup species differences were plotted as bar charts. The metagenomeSeq analysis was performed using R's metagenomeSeq package, and the permutation test was performed at each classification level to obtain the p value, which was then analyzed by STAMP (v2.1.3) and plotted as a graph of intergroup species differences. The p-value was then corrected using the Benjamini and Hochberg false discovery rate method to obtain the q-value, and the relative abundance distributions of the dissimilar species between groups were plotted in box plots for each level with statistical significance (p value / q value < 0.05).

The compositions described in Embodiments 2 and 3 were tested by the experimental method described in Embodiment 4 with the test results as follows.
1. Effects of the Composition of Present invention on Daily Food, Water and Energy Intake and Body Weight of Obesity Rats.
In this experiment, the obesity-induced rats were fed with high-fat diet, and according to the results in Table 2 below, the daily energy intake of the other groups after high-fat diet was significantly increased compared with the NOR group (p < 0.05). With no difference in mean starting body weight, the results of weight gain during the animal experiment showed that the weight gain of rats induced by HF was significantly increased (p<0.05), but the weight gain was significantly reduced by feeding different compositions (RL, RH and Msol) (p<0.05).

**Table 2. Effects of Feeding RL, RH and Msol on Body Weight Changes of High-Fat diet Rats**

| Groups | Weight | Weight | Weight gain (g) |
|---|---|---|---|
| | 0 week (g) | 8 week (g) | |
| NOR | 295.8± 6.0^{a} | 510.1 ± 26.7^{a} | 214.4± 27.78^{a} |
| HF | 296.6± 8.6^{a} | 584.5 ± 17.1^{c} | 287.9± 19.4^{c} |
| RL | 289.1:1: 6.9^{a} | 527.3 ± 22.6^{ab} | 238.1± 18.8^{ab} |
| RH | 290.00± 9.1^{a} | 548.0 ± 21.5^{b} | 258.0± 17.4^{b} |
| Msol | 291.4 ± 10.0^{a} | 512.1 ± 31.1^{a} | 220.8 ± 38.8^{a} |

2. The results in Table 3 indicated that the HF group caused an abnormal increase in liver weight compared to the NOR group, a phenomenon that might imply that a daily high-fat diet would result in abnormal liver tissues, however, this condition was significantly improved by feeding the RL, RH, and Msol groups, which resulted in a significant decrease in liver weight (p < 0.05), as well as a reduction in the liver-to-body-weight ratio.

**Table 3 Effects of Feeding RL, RH and Msol on Liver Weight and Liver/Body Weight of High-Fat diet Rats**

| Groups | Liver weight (g) | Weight before Sacrifice (g) | Liver Weight/Body Weight (%) |
|---|---|---|---|
| NOR | 13.69 0.81^{a} | 489.5±27.1^{a} | 2.71 ± 0.11^{a} |
| HF | 22.72 2.11^{d} | 568.8± 17.6^{c} | 3.94 ± 0.40^{d} |
| RL | 18.12 2.00^{bc} | 512.9± 24.7^{ab} | 3.39 ± 0.24^{bc} |
| RH | 20.06 1.39^{c} | 524.0± 20.6^{b} | 3.66 ± 0.27^{cd} |
| Msol | 16.84 2.31^{b} | 488.5± 31.7^{a} | 3.28 ± 0.37^{b} |

3. The Effect of the Composition of the Present Invention on the Regulation of Body Fat of Obesity Rats
From the results in Table 4, we could see that the body fat mass and body fat percentage of obesity rats induced by high-fat diet increased significantly (p < 0.05) compared with those of the NOR group, and the body fat mass and body fat percentage of obesity rats were significantly reduced by feeding of RL, RH, and Msol (p < 0.05). This result showed that both red yeast rice containing Monascinol and Monascinol had the effect of reducing body fat.

**Table 4. Effects of Feeding RL, RH and Msol on Body fat Weight and Body Fat Percentage of High-Fat diet Rats**

| Groups | Fat weight (g) | Weight before sacrifice (g) | Fat/body weight (%) |
|---|---|---|---|
| NOR | 10.86 1.84^{a} | 489.5± 27.1^{a} | 2.12 ± 0.28^{a} |
| HF | 25.57 4.48^{c} | 568.8± 17.6^{c} | 4.43 ± 0.70^{c} |
| RL | 18.32 1.94^{b} | 512.9± 24.7^{ab} | 3.36 ± 0.30^{b} |
| RH | 18.82 5.31^{b} | 524.0± 20.6^{b} | 3.32 ± 0.71^{b} |
| Msol | 17.95 3.55^{b} | 488.5± 31.7^{a} | 3.35 ± 0.55^{b} |

4. Effect of the Composition of the Present Invention on Lipid regulation of Obesity Rats
The high fat-diet increased TC and TG concentrations in the serum and liver, and caused lipid accumulation in the liver resulting in damage. As shown in Table 5, the serum concentrations of TC and TG induced by high-fat diet in HF group were significantly higher than those in NOR group (p<0.05), while the feeding of RL and RH significantly reduced the TC concentrations (p<0.05), and Msol, a pure substance, also reduced the serum concentrations of TC induced by high-fat diet (p<0.05). This shows that Msol is a functional component of red yeast rice to reduce TC. In terms of serum TG concentration, the HF group significantly increased the TG concentration compared with the NOR group (p<0.05), whereas the red yeast rice groups at all doses significantly decreased the TG concentration (p<0.05), and the Msol group also had a significant reduction effect (p<0.05).

**Table 5. Effects of Feeding RL, RH and Msol on TC and TG in Serum of High-Fat diet Rats**

| Groups | TC (mg/dL) | TG (mg/dL) |
|---|---|---|
| NOR | 52.2± 7.3^{a} | 55.3 ± 6.0^{a} |
| HF | 75.3± 5.3^{c} | 77.0 ± 7.1^{b} |
| RL | 53.3± 6.8^{a} | 59.4 ± 6.8^{a} |
| RH | 54.7± 5.6^{a} | 56.9 ± 5.4^{a} |
| Msol | 65.0± 10.4^{b} | 61.7 ± 10.7^{a} |

5. Effect of the Composition of the Present Invention composition on the Regulation of Hepatic Lipids of Obesity Rats
The results of liver lipid analysis in Table 6 showed that the HF group increased the concentration of TC and TG in the liver (p < 0.05), the feeding of low-dose red yeast rice significantly reduced the accumulation of TC and TG in the liver caused by the high-fat diet (p < 0.05), and the RL, RH and Msol were effective in reducing the hepatic TC levels. In terms of reducing TG content, RL, RH and Msol were significantly different from the HF group (p < 0.05). The above results show that red yeast rice has the potential to reduce the accumulation of TC and TG in the liver caused by obesity, and has the potential to improve the occurrence of fatty liver. Msol is the functional component in red yeast rice that reduces liver lipids.

**Table 6. Effects of Feeding RL, RH and Msol on TC and TG Concentrations in the Liver of High-Fat diet Rats.**

| Groups | Liver TG (mg/g) | Liver TC (mg/g) |
|---|---|---|
| NOR | 19.57 4.04^{b} | 6.25 ± 0.42^{b} |
| HF | 40.14 2.79^{c} | 7.91 ± 0.42^{c} |
| RL | 17.68 2.77^{ab} | 5.34 ± 0.68^{a} |
| RH | 17.06 2.94^{ab} | 4.89 ± 0.68^{a} |
| Msol | 14.40 ± 1.57^{a} | 5.05 ± 0.54^{a} |

6. Effect of the Composition of the Present Invention on Fecal Lipid Content in Obesity Rats
The analysis of TC and TG levels in feces (Table 7) showed that TC and TG levels in feces induced by high-fat diet were significantly higher than those in the NOR group (p < 0.05). Feeding of RL, RH promoted the excretion of TC in the body, and the Msol group also significantly promoted the excretion of TC significantly (p < 0.05). The results of fecal TG content showed that RL, RH and Msol were effective in reducing the accumulation of TG in the body. Therefore, it was implied that red yeast rice and Msol were able to enhance the TG metabolism in the body in exchange for decreasing the accumulation in the liver, which might also indicate that red yeast rice and Msol could promote the reduction of body fat by eliminating lipids.

**Table 7. Effects of Feeding RL, RH and Msol on TC and TG Concentrations in the Feces of High-Fat diet Rats**

| Groups | Feces TG (mg/g) | Feces TC (mg/g) |
|---|---|---|
| NOR | 18.25 3.25^{a} | 15.93 ± 1.47^{a} |
| HF | 26.45 3.35^{b} | 38.03 ± 2.33^{b} |
| RL | 58.56 5.41^{c} | 53.97 ± 8.59^{d} |
| RH | 63.88 5.01^{d} | 51.22 ± 4.13^{d} |
| Msol | 61.27 4.80^{cd} | 46.22 ± 4.72^{c} |

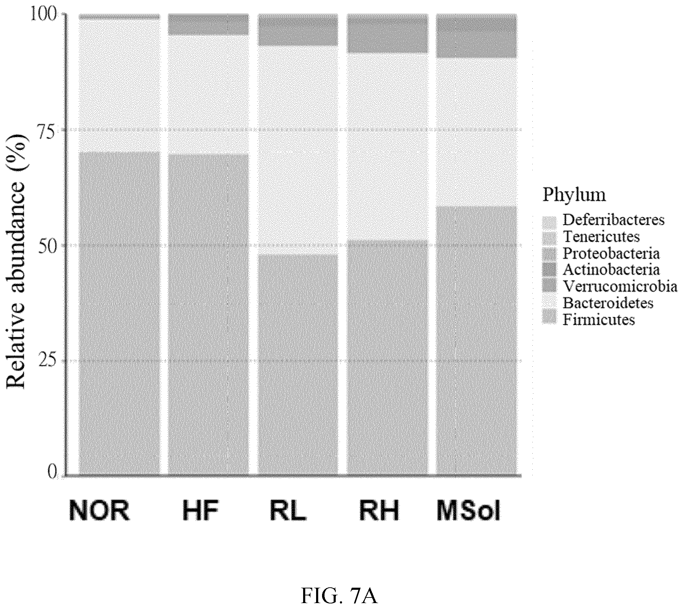

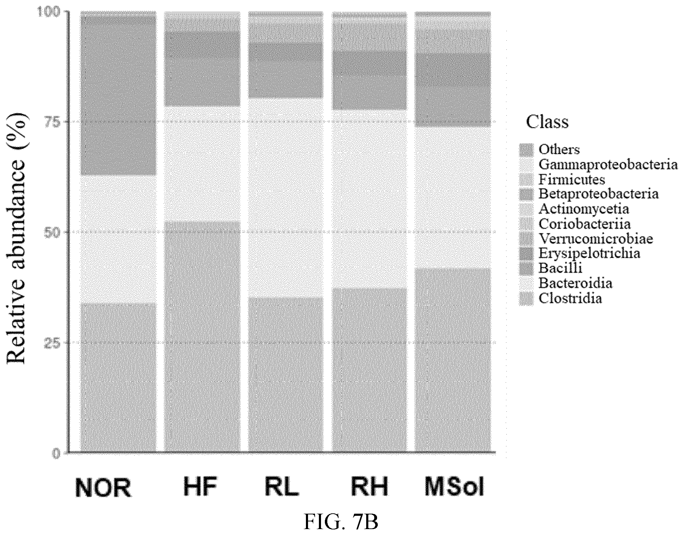

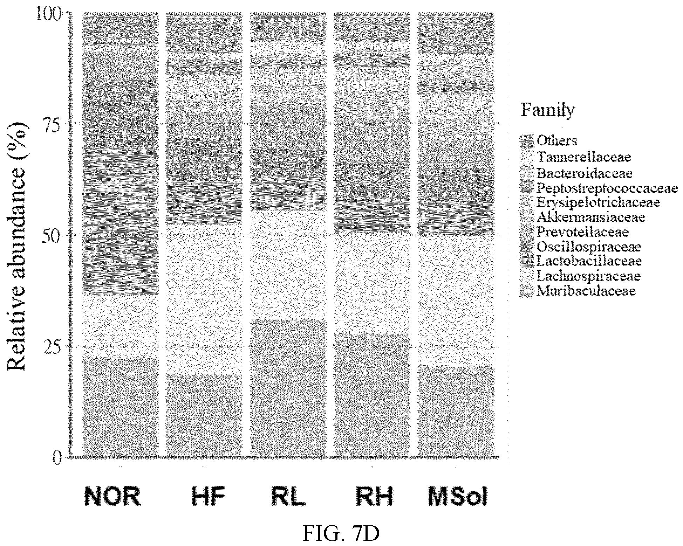

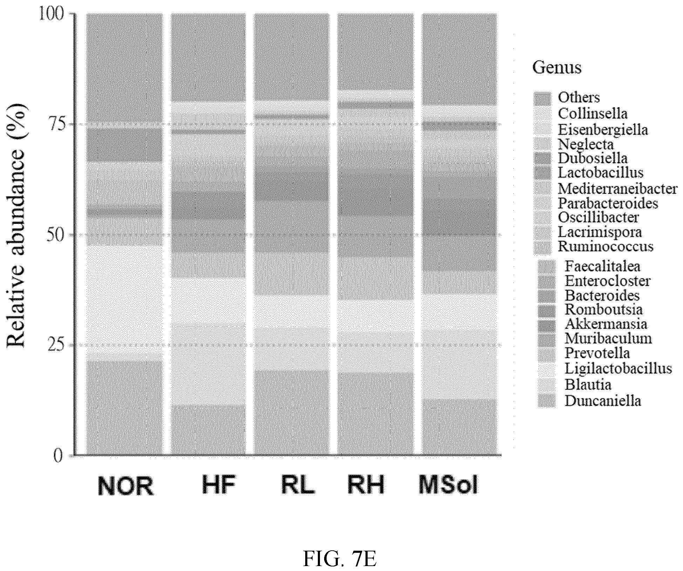

7. Effect of Feeding the Red Yeast Rice and Monascinol of the Present Invention on the Gut Microbiota of High-Fat Diet Rats
Firmicutes and Bacteroidetes are most abundant in the normal intestinal tract, with a combined percentage of more than 80%, and the abundance ratio of Firmicutes to Bacteroidetes (F/B ratio) is a factor that has been used in recent years in microbiome research to measure the risk of specific diseases. Early literature suggested that the relatively high proportion of Firmicutes was related to the obesity of mammals (including rats and humans). FIGS. 7A~7F show the effects of feeding the red yeast rice and Monascinol of the present invention on the gut microbiota of high-fat diet rats, which are shown in the relative abundance plots based on the phylum, class, order, family, genus and species levels of ASV. In addition, as shown in the bacterial abundance analysis in FIG. 7A, the classification of the phylum was mainly in the seven phyla: Bacteroidetes, Proteobacteria, Verrucomicrobia, Deferribacteres, Actinobacteria, Firmicutes and Tenericutes, with the highest abundance of Firmicutes, followed by Bacteroidetes. When a rat on high-fat diet was fed with the red yeast rice and Monascinol of the present invention, there was a decrease in the abundance of Firmicutes and an increase in Bacteroidetes (i.e., a decrease in the F/B ratio), suggesting that the red yeast rice of the present invention was effective in decreasing obesity-associated disease risk factors.

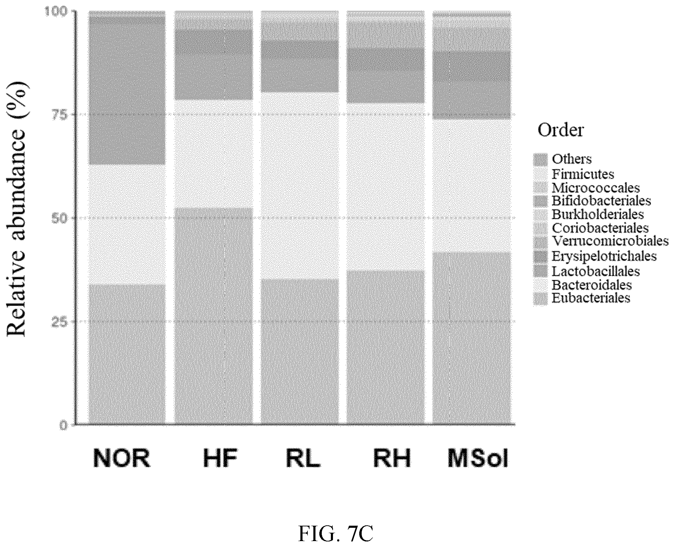

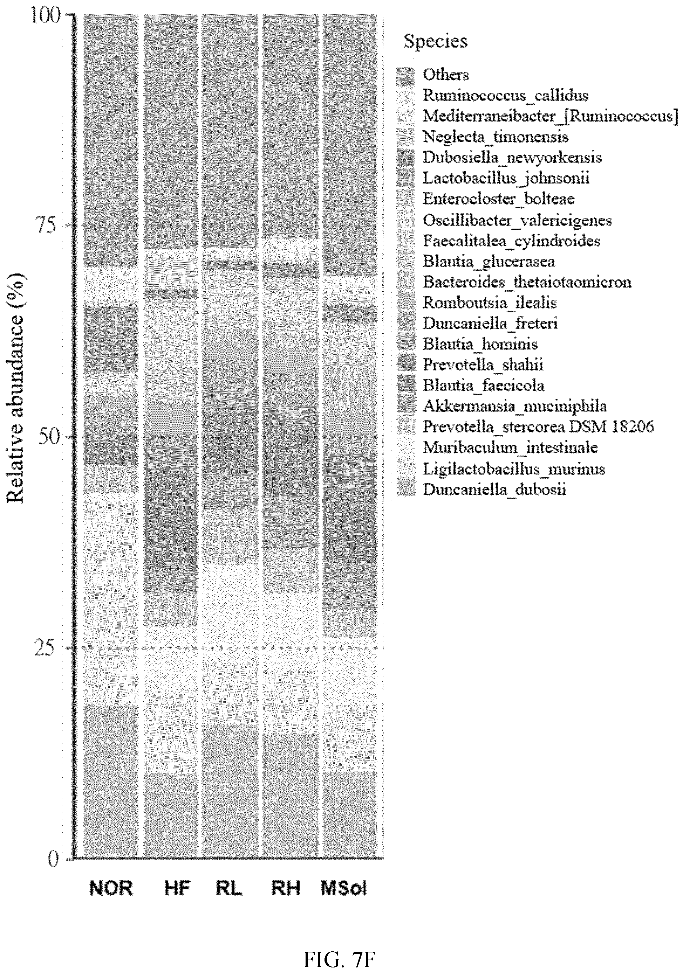

Akkermansia muciniphila, a member of Verrucomicrobia, has been shown to be a beneficial intestinal bacterium that can improve metabolic disorders, blood glucose stabilization, and insulin sensitivity in high-fat diet induced obesity mice, as well as inhibit obesity-induced mild gastrointestinal inflammation and inhibit the manifestation of adipose-associated factors, reduce adipogenesis, and prevent hepatic steatosis, which is conducive to the maintenance of intestinal health. FIG. 7C shows that feeding the test substances including red yeast rice containing Monascinol and Monascinol increased the intestinal abundance of Verrucomicrobiales, and FIG. 7F shows that red yeast rice and Monascinol increased the abundance of A. muciniphila in high-fat diet-induced obesity rats to improve the lipid metabolism of obesity rats induced by a high-fat diet.

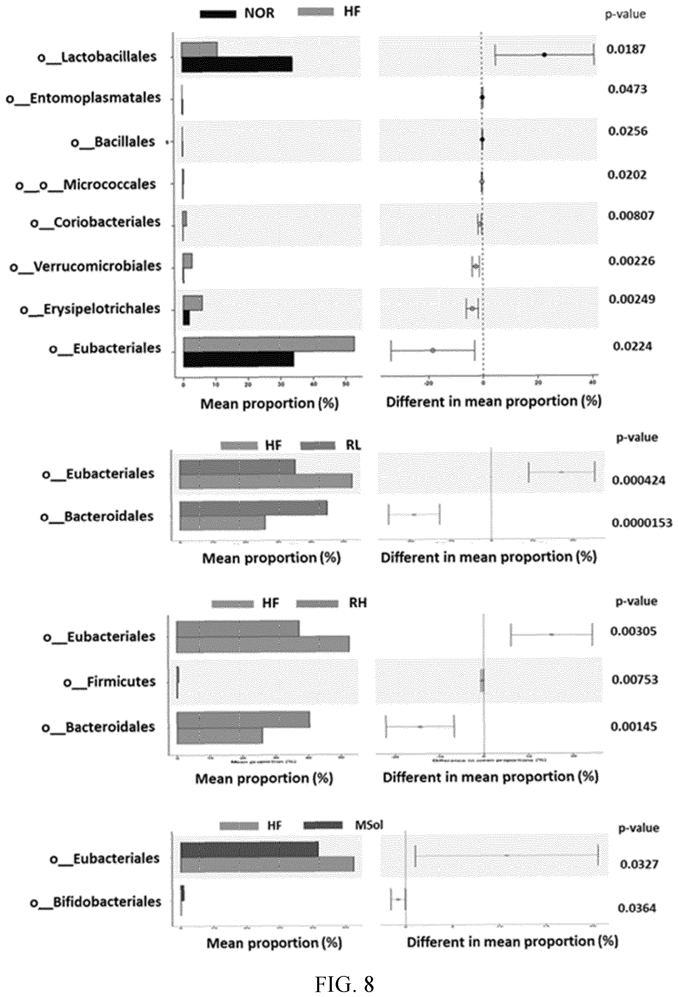

The Bifidobacteriales of Actinobacteria are recognized as an important component of healthy human intestinal probiotics and have many beneficial properties including maintenance of gut microbiota homeostasis, improved lactose digestibility, anticancer activity, reduced serum cholesterol, and improved calcium absorption. As shown in FIG. 7C, feeding the red yeast rice and Monascinol of the present invention increased the bacterial abundance of Bifidobacteriales in the intestine. In addition, compared with the normal diet (NOR group) as shown in FIG. 8, the high-fat dieted animals (HF group) significantly increased the intestinal abundance of the harmful bacteria Eubacteriales (35.07% to 50.14%) and decreased the abundance of the beneficial bacteria Lactobacillales (36.94% to 10.66%) (p<0.05). Compared with the HF group (50.14%), the feeding of red yeast rice and Monascinol of the present invention significantly reduced the abundance of Eubacteriales (36.91%, 37.66%, and 43.21%), whereas in terms of the abundance of the Bacteroidales, the feeding of either low dose (RL) or high dose (RH) red yeast rice fermentation products significantly increased the bacterial abundance of the Bacteroidales (p<0.05). In the analysis of gut microbiota composition at the genus level, the abundance of beneficial bacteria such as Ligilactobacillus and Lactobacillales in the red yeast rice fermentation product experimental groups was significantly higher than that in the HF group (p<0.05).

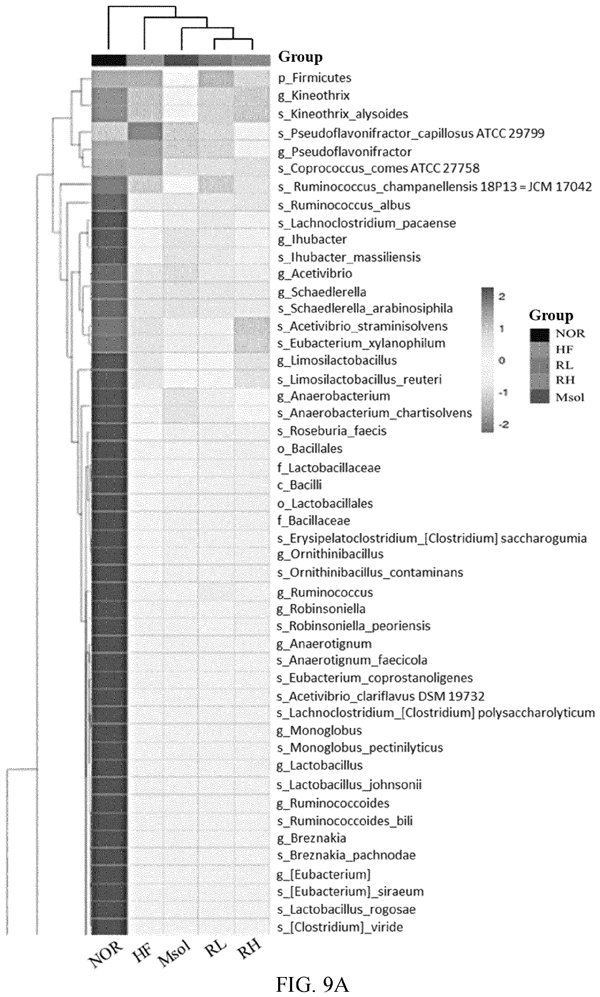

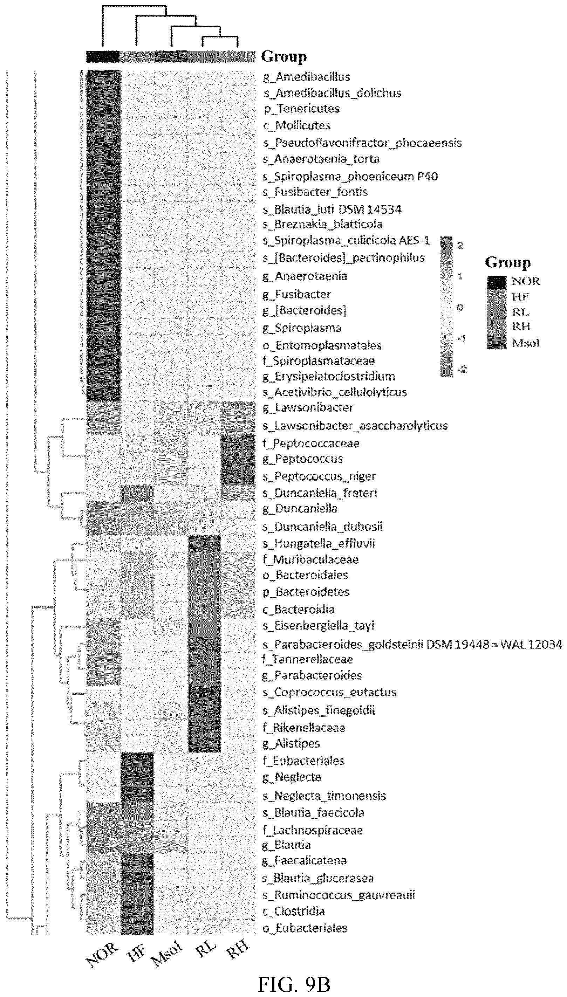

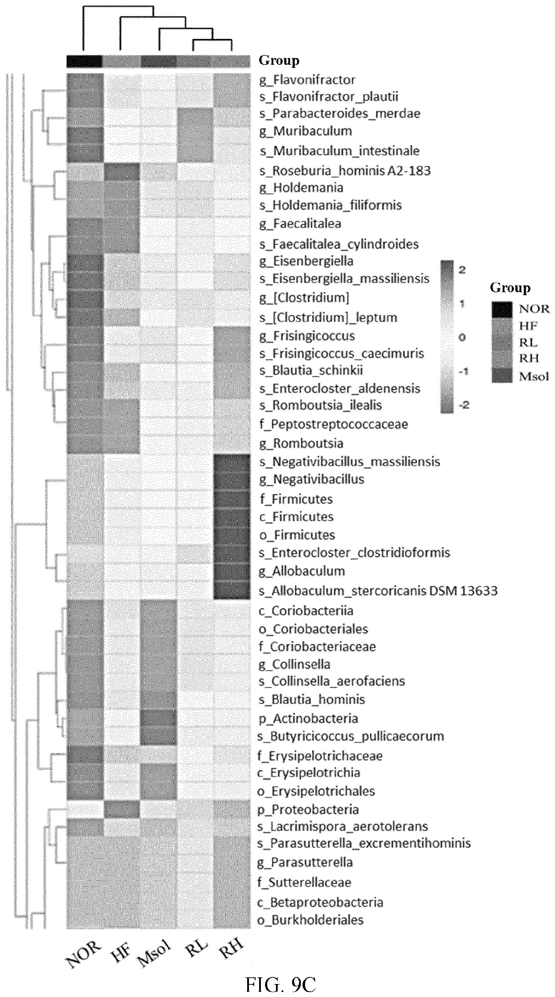

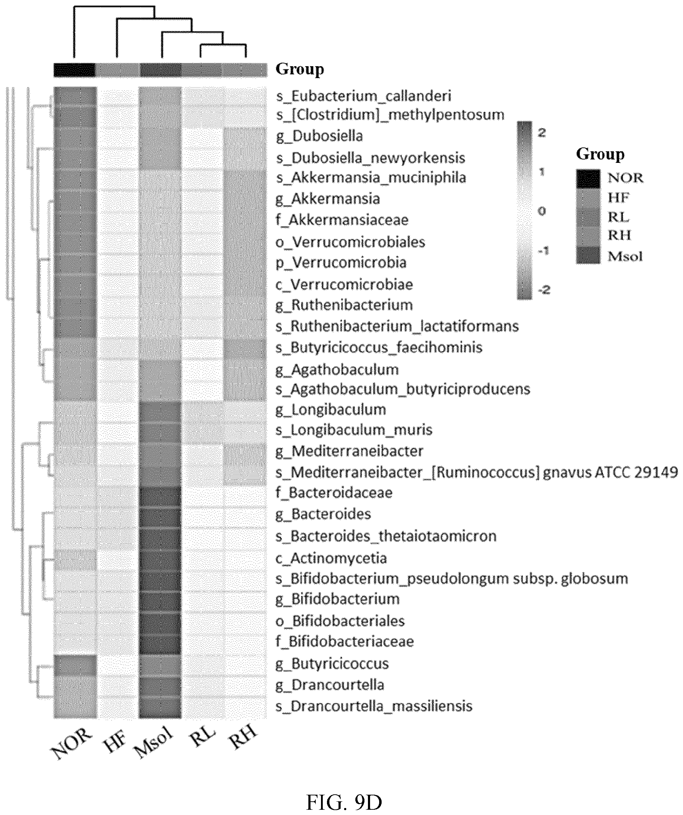

The relative abundance hotspot plots of bacterial aggregation in each experimental group (as shown in FIGS. 9A~9D) showed the flora composition of each experimental group, the flora composition of the HF group induced by the high-fat diet was different from that of the NOR group, and the feeding of the red yeast rice of the present invention revealed that the red yeast rice did not restore the gut microbiota composition to that of the NOR, but rather increased the beneficial bacterial abundance to improve the flora composition caused by high-fat diet. For example: Feeding red yeast rice and Monascinol can increase the intestinal abundance of A. muciniphila, as mentioned above, and also increase the intestinal abundance of Dubosiella newyorkensis, which has been applied for a U.S. patent as a probiotic for the treatment of obesity, diabetes, and other metabolic syndromes and immune-related gastrointestinal diseases, etc. Bacteroides thetaiotaomicron is one of the probiotics that are symbiotic with human intestines. B. thetaiotaomicron is highly related to carbohydrate, starch metabolism and polysaccharide utilization, which are not only involved in nutrient metabolism, but also have different positive effects on intestinal ecosystems, intestinal immune system, and intestinal gene expression. The positive effect of red yeast rice on B. thetaiotaomicron was also observed in the intestines, and the effect of Monascinol was the most significant, confirming that Monascinol regulates B. thetaiotaomicron as a major effector and has the potential to improve the stability of the intestinal system.

In summation of the results given above, high-fat diet will change the composition of gut microbiota of normal rats, thereby affecting lipid metabolism in the body and exacerbating the risk of obesity. In the present invention, the red yeast rice and Monascinol can improve the composition of gut microbiota to reduce the abundance ratio of Firmicutes and Bacteroidetes, increases the bacterial abundance of beneficial intestinal bacteria such as Verrucomicrobiales and Bifidobacteriales, and reduces obesity-related flora, thereby regulating lipid metabolism in the body and reducing the accumulation of serum, liver cholesterol and triglyceride, and increasing the excretion fecal lipids, and achieving the effect of reducing body fats.

The present invention uses different test substances (compositions) including RL, RH and Msol to evaluate the effect of improving composition of gut microbiota and body fats in 8-week high-fat diet-induced obesity rats, and the following conclusions are summarized from the above embodiments and test results: 1. In the present invention, the red yeast rice group (RL, RH) contains Monascinol as an ingredient. The red yeast rice powder (RL, RH) has been shown to have significant effects on weight change, liver weight and liver/body weight, body fat weight and body fat percentage, and serum and liver TC, TG concentrations at a low dose of 0.5 g per person per day. This dose is very low and has been shown to be effective, and this substance has been shown to be quite progressive in reducing body fats.
2. In the present invention, Monascinol is found to have a significant effect on the change of body weight, liver weight and liver/body weight, body fat weight and body fat percentage, and serum and liver TC and TG activity, thus achieving a significant reduction in body fat at low levels. The dose of Monascinol is 3 mg per person per day, which is a very low and effective amount and has been shown to be progressive in reducing body fats.
3. High-fat diet will change the composition of gut microbiota of normal rats, thereby affecting lipid metabolism in the body and exacerbating the risk of obesity. In the present invention, red yeast rice and Monascinol can increase the bacterial abundance of beneficial intestinal bacteria such as Verrucomicrobiales and Bifidobacteriales to reduce the ratio of Firmicutes to Bacteroidetes and reduce obesity-related flora, thereby regulating lipid metabolism in the body, reducing the accumulation of serum cholesterol, triglycerides, liver cholesterol and triglycerides, increasing the excretion of fecal lipids, and achieving the effect of reducing body fat and weight.

The description above has already completely and clearly described the novel Monascus pilosus of the present invention for the purpose of losing weight and improving the composition of gut microbiota and the use of the Monascus pilosus. It is to be understood that the above detailed description is a specific description of possible embodiments of the present invention, but there is no intent to limit the scope of the present invention by those embodiments. On the contrary, it is intended to cover various modifications and similar arrangements included within the spirit and scope of the appended claims which are to be accorded with the broadest interpretation so as to encompass all such modifications and similar arrangements.

### Deposit of Microorganisms

The Monascus pilosus of the present invention was deposited at the National Collections of Industrial, Food and Marine Bacteria (NCIMB Ltd) under the Accession Number of NCIMB 44103 on January 12, 2023.

## Claims

1. A Monascus pilosus, deposited at the National Collections of Industrial, Food and Marine Bacteria (NCIMB Ltd) under the Accession Number NCIMB 44103.

2. A use of a Monascus pilosus in the manufacture of a composition for losing weight and improving gut microbiota composition, wherein the Monascus pilosus may be the Monascus pilosus deposited at the National Collections of Industrial, Food and Marine Bacteria (NCIMB Ltd) under the Accession Number NCIMB 44103.

3. A composition for losing weight and improving gut microbiota composition, comprising an active ingredient with an effective amount, wherein the active ingredient is extracted from a Monascus pilosus fermented product, the Monascus pilosus fermented product is prepared by fermenting a base material by a Monascus pilosus, and the Monascus pilosus may be the Monascus pilosus deposited at the National Collections of Industrial, Food and Marine Bacteria (NCIMB Ltd) under the Accession Number NCIMB 44103.

4. The composition according to claim 3, wherein the composition is capable of enhancing bacterial abundance of Bacteroidales, and lowering ratio of Firmicutes to Bacteroidetes.

5. The composition according to claim 3, wherein the composition is capable of enhancing abundance of beneficial intestinal flora and changing gut microbiota composition.

6. The composition according to claim 3, wherein the active ingredient comprises at least one selected from the group consisting of Monascinol, Ankaflavin and Monascin.

7. The composition according to claim 3, wherein the base material is a rice, dioscoreae rhizoma, or mixture of related carbohydrates.

8. The composition according to claim 3, wherein the effective amount when used alone is at least 0.75 mg to 12 mg of the active ingredient per day for adults.

9. A composition for losing weight and improving gut microbiota composition, comprising a Monascus pilosus fermented product with an effective amount, wherein the Monascus pilosus fermented product is prepared by fermenting a base material by a Monascus pilosus, and the Monascus pilosus may be the Monascus pilosus deposited at the National Collections of Industrial, Food and Marine Bacteria (NCIMB Ltd) under the Accession Number NCIMB 44103.

10. The composition according to claim 9, wherein the composition is capable of enhancing bacterial abundance of Bacteroidales, and lowering ratio of Firmicutes to Bacteroidetes.

11. The composition according to claim 9, wherein the composition is capable of enhancing abundance of beneficial intestinal flora and changing gut microbiota composition.

12. The composition according to claim 9, wherein the base material is a rice, dioscoreae rhizoma, or mixture of related carbohydrates.

13. The composition according to claim 9, wherein the effective amount is at least 0.5 g per day for adults and containing 1.5 mg of monascinol.

14. The composition according to claim 9, wherein the Monascus pilosus fermented product comprises at least one active ingredient, and the active ingredient is at least one selected from the group consisting of Monascinol, Ankaflavin and Monascin.

15. The composition according to claim 9, wherein the composition is a food composition, a pharmaceutical composition, a feed composition, a nutritional supplement composition, a dietary supplement composition, or a food additive composition.
